# EUROPEAN PATENT SPECIFICATION

(11) **EP 2 838 385 B1**
(45) Date of publication and mention of the grant of the patent: **20.11.2019**
(21) Application number: 12874753.2
(22) Date of filing: 18.04.2012
(51) Int. Cl.: A24F 47/00

(54) **ELECTRONIC CIGARETTE**
ELEKTRONISCHE ZIGARETTE
CIGARETTE ÉLECTRONIQUE

(43) Date of publication of application: 25.02.2015
(73) Proprietor: Fontem Holdings 1 B.V., 1083 HN Amsterdam (NL)
(72) Inventor: HON, Lik, North Point Hong Kong (CN)
(74) Representative: Gulde & Partner
(86) International application number: PCT/CN2012/000530
(87) International publication number: WO 2013/155645

(56) References cited:
- EP-A1- 2 404 515
- WO-A1-2009/155734
- WO-A1-2009/155734
- CN-A- 101 228 969
- CN-A- 101 228 969
- CN-A- 102 160 906
- CN-Y- 201 379 072
- CN-Y- 201 379 072
- US-B2- 8 851 068

## Description

### BACKGROUND

Electronic cigarettes are increasingly used by smokers as a substitute for real tobacco cigarettes. In general, electronic cigarettes use a wire coil heater to vaporize liquid nicotine, or other liquid substances. The user's inhalation on a mouthpiece may be detected by a sensor, causing an electronic circuit to supply electrical current from a battery to the heater. The liquid contacts the wire coil heater, which creates the vapor or mist. The user's inhalation typically also draws ambient air into one or more inlets in the electronic cigarette housing. The vapor is entrained in the air flow moving through the housing and is inhaled by the user.

Electronic cigarettes have many advantages over real tobacco cigarettes. Initially, the risks of lung cancer associated with real tobacco cigarettes is largely avoided, as the tar and other chemicals in tobacco linked to lung cancer are simply not present in an electronic cigarette. Electronic cigarettes generate vapor or mist, and not smoke. Consequently, there is no comparable second-hand smoke problem with use of electronic cigarettes. In addition, since there is no burning material in electronic cigarettes, the risk of fire is eliminated.

Many electronic cigarette designs have been proposed and used, with varying degrees of success. Existing designs though have various disadvantages, including short life, poor atomization, nonuniform vapor caused by different sizes of liquid drops, and overheated vapor. For instance, CN 101228969 A discloses a liquid guide means in contact with a liquid storage component such that liquid sprays into an atomization chamber onto a heater for atomization. Both US 8 851 068 B2 and EP 2 404 515 A1 disclose a liquid permeating means to guide liquid directly to a heater. Accordingly, there is a need for an improved electronic cigarette.

### SUMMARY OF THE INVENTION

An electronic cigarette according to the present invention provides significant improvements over existing designs. In this electronic cigarette, as defined in claim 1, a mesh element is in contact with the liquid storage. A heater is spaced apart from the mesh element and positioned such that liquid does not come into direct contact with the heater and air which flows through the mesh element is heated. The heated air vaporizes the liquid in or on the mesh. The vapor is inhaled by the user.

In another aspect, the heater may be positioned within a heater housing having an air passageway aligned with a central opening extending through the liquid storage. Alternatively, an annular flow path around the outside of the liquid storage may be used.

The electronic cigarette may include a battery in the housing electrically connected to a flow sensor, a circuit board and the heater. A flow path through the housing may be formed via one or more inlets in the housing, a passageway containing the heater, and a central opening extending through the liquid storage to an outlet.

In a separate aspect, a method of vaporizing a liquid in an electronic cigarette includes conducting liquid from a liquid storage to a mesh element. Electric current is supplied to a heater, optionally in response to sensing inhalation on the outlet or mouthpiece of the electronic cigarette. The heater heats air and the heated air is conducted through the mesh element, with the heated air vaporizing liquid on or in the mesh element. The vaporized liquid is entrained with the heated air and may then flow through or around the liquid storage to the mouthpiece.

Other and further objects and advantages will become apparent from the following detailed description which is provide by way of example.

### BRIEF DESCRIPTION OF THE DRAWINGS

In the drawings, the same reference number indicates the same element in each of the views.
Fig. 1 is a section view of an electronic cigarette according to a first embodiment of the invention.
Fig. 2 is an enlarged detail view of components of the electronic cigarette shown in Fig. 1.
Fig. 3 is a schematic section view of an alternative embodiment.
Fig. 4 is a perspective view of a component of the design shown in Fig. 3.

### DETAILED DESCRIPTION

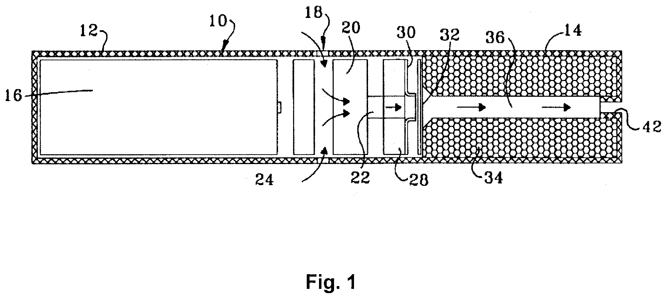

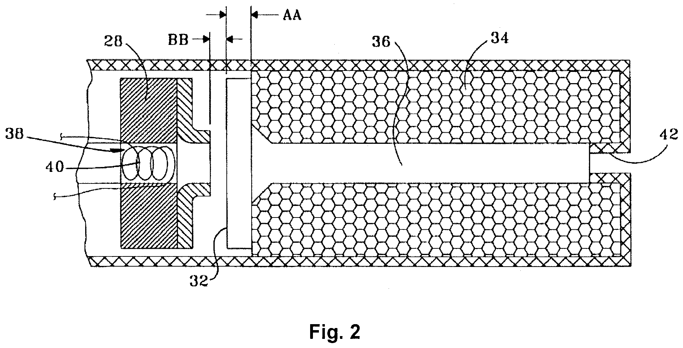

Turning now in detail to the drawings, as shown in Figs. 1 and 2, and electronic cigarette has a housing 10 which optionally may be provided with a front section 12 attached to a back section 14 via screw threads or other attachment. A battery 16 and a circuit board 24 may be contained within the front section, with the circuit board electrically connected to a flow sensor 20 and to a heater coil 40. A liquid storage 34 is contained within the back section 14 of the housing 10. The liquid storage 34 may be a fiber material, provided loose in bulk directly into the back section 14 of the housing, or it may be provided in or as part of a separate component or cartridge. The liquid storage may contain liquid nicotine, or another liquid for vaporization and inhalation. Other materials such as foam or porous metals or ceramics may optionally be used as the liquid storage 34.

The heater coil 40 may be positioned within a passageway 38 extending through a heater support 28. The heater housing, for example, a ceramic material, is fixed in place within the housing. An optional collector 30 may be attached to the back end of the heater support 28, with the passageway also extending centrally through the collector 30. The collector 30, if used, may be made of Silastic® silicone elastomers, or other high temperature inert silicon elastomers or plastic materials.

A mesh element or screen 32 on the front end of the liquid storage 34 is spaced slightly apart from the back end of the collector 30, by a dimension BB ranging from about 0.5 to 2 or 4 mm, and typically about 1 mm. The mesh 32 may be fiberglass, or other porous material, which the liquid in the liquid storage, such as liquid nicotine, can wick onto or through. The mesh 32 may have a thickness or dimension AA in Fig. 2 ranging from about 0.1 to 2 mm, 0.2 to 1mm, or 0.3 to 0.6 mm, with a 4 mm thickness typical.

An opening 36 extends from the mesh 32 centrally through the liquid storage 34 to an outlet 42 at the back end of the housing 10. A flow path may be formed through the housing 10 via one or more inlets 18, a through opening in the sensor 20, the flow tube 22, the passageway 38 and the opening 36 leading to the outlet 42. Except as specified, the positions of the elements shown in the drawings is not critical, and the elements may be rearranged as needed or desired.

Referring still to Figs. 1 and 2, in an example of use, the user inhales on the outlet 42. The sensor 20 detects the inhalation and supplies electrical current to the heater coil 40. Air is drawn into the flow path in the housing through the inlets 18. The flowing air passes through the passageway 38 and is heated by the heater coil 40. The amount of heating may vary by design. Air temperatures of 200 to 300 °C at the exit of the passageway, as one example, may be used by adjusting the power of the heater and the air flow characteristics through or past the heater. The collector 30, if used, may help to collect and direct the heated air to the mesh 32. The collector 30 may also be used to space the heater coil 40 and the heater support 28 apart from the mesh 32. The collector may optionally be made part of the heater support 28.

The mesh 32 is provided as a thin sheet or layer, and has a sufficiently open structure, so that the heated air can pass through without excessive flow resistance. The mesh 32 may be a sheet or layer of loose fiberglass, fiberglass fabric or similar material that can wick and hold liquid on the surface of the fibers, and/or in the gaps between the fibers, and also allow air to flow through. A heat resistant foam material may alternatively be used in place of the mesh.

The heated air flows through the mesh 32. This heats liquid in or on the mesh, which atomizes or vaporizes the liquid. The vapor is entrained in the heated air, which continues flowing from the mesh 32 through the opening 36 and the outlet 42, with the mixture of air and vapor inhaled by the user. The heated air may cool considerably as it passes through the mesh 32 and the opening 36, so that the user inhales air from the outlet at a comfortable temperature of e.g., 25 to 50 °C.

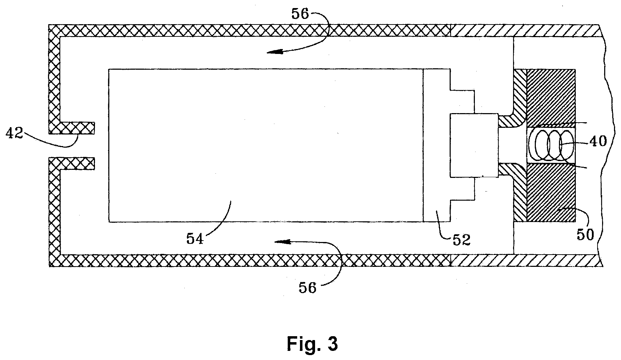

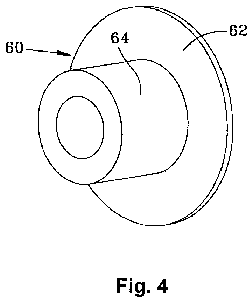

Figs. 3 and 4 show an alternative design having a similar operation, but with the airflow path extending around the outside of a liquid storage element 54, rather than through the liquid storage, as in Figs. 1 and 2. In the alternative design of Figs. 3 and 4, the liquid storage is surrounded by an annular passage 56, leading from a woven or mesh tube 52 to the outlet 42. As also shown in Figs. 3 and 4, the mesh tube 52 has a plate section 62 in contact with the liquid storage. A neck section 64 of the mesh tube 52 extends from the plate section 62 towards the heater 50. Liquid in the liquid storage 54 wicks through the plate section and into the neck section 64. Heated air diffusing radially outwardly through the neck section vaporizes the liquid creating a mist or vapor, which is drawn through the flow path 56 and inhaled by the user. The woven tube 62 may be produced by twill weave, and then cut with a hot blade, to prevent unraveling of cut end. Of course, the thin flat mesh component 32 shown in Fig. 2 may also be used in an embodiment having the annular passage 56 as shown in Fig. 3.

In the designs described above the liquid does not come into direct contact with the heater coil. This avoids the loss of heating efficiency resulting from deposits and liquid residue collecting on the heater coil 40. It also allows for longer heater coil life, as thermal shock to heater coil, and corrosion are reduced. Vaporization is also improved because the liquid is vaporized at lower temperatures. The heater coil itself may operate at temperatures in the range of 500 °C. This can cause chemical changes in the liquid as it is vaporized. By avoiding contact between the heater coil and the liquid, and by vaporizing the liquid using heated air, chemical changes occurring during vaporization may be reduced.

In addition, since the heater coil 40 does not come into contact with the liquid, the heater coil may be plated with corrosion resistant materials, such as silver or nickel-chromium. Use of these types of materials, which would be degraded if contacted by the liquid, prolongs the life of the heater coil. Since the life of the heater coil can be much longer, the heater coil can be made as a reusable component, rather than be a disposable item as is common with existing designs. This allows for reduced costs.

With existing known electronic cigarettes, the heating device or coil must heat the nicotine liquid first, before the liquid can be vaporized. The electronic cigarette according to the present invention omits this initial step, as the heater coil 40 heats air, and not liquid. Consequently, the new designs described here also achieve faster vaporization in comparison to known designs.

Thus, designs according to the invention have been shown and described. Various changes and substitutions may of course be made without departing from the scope of the invention, as defined by the appended claims.

## Claims

1. An electronic cigarette comprising:
a housing (10) having one or more inlets (18) and an outlet (42);
a liquid storage (34, 54) within the housing (10);
a mesh element (32, 52) in contact with the liquid storage (34, 54); and
a heater (40) spaced apart from the mesh element (32, 52) and positioned to heat air which flows through the mesh element (32, 52);
wherein the mesh element (32, 52) is positioned between the heater (40) and the liquid storage (34, 54) within a flowpath formed, in use, in the housing (10); **characterized in that**
the flowpath is formed, in use, through the housing (10) via the one or more inlets (18), a passageway comprising the heater (40), and an opening (36, 56) leading to the outlet (42);
the mesh element is made of a porous material, the porous material being configured to wick liquid stored in the liquid storage (34, 54), when in use; and
wherein the mesh element (32, 52) is further configured to allow, in use, liquid being wicked on or in the mesh element (32, 52) to be vaporized by heated air conducted through the mesh element (32, 52).

2. The electronic cigarette of claim 1 with the heater (40) within a heater support (28) having an air passageway (38) aligned with a central opening (36) extending through the liquid storage (34).

3. The electronic cigarette of claim 2 further including a collector (30) on the heater support (28), with the collector spaced apart from the mesh element (32) by less than 4 mm.

4. The electronic cigarette of claim 2 with the heater support (28) having tubular front end contacting the mesh element (32).

5. The electronic cigarette of claim 1 further comprising an annular flow path (56) around the outside of the liquid storage (54).

6. The electronic cigarette of claim 1 with the mesh element (32, 52) comprising a fiber material having a thickness less than 2 mm.

7. The electronic cigarette of claim 1 further comprising a battery (16) in the housing (10) electrically connected to a flow sensor (20), a circuit board (24) and the heater coil (40).

8. The electronic cigarette of claim 1, wherein the opening is a central opening (36)extending through the liquid storage (34) to an outlet (42).

9. An electronic cigarette according to claim 1, further comprising:
a battery (16) electrically connected to a flow sensor (120) and an electronic circuit in the housing (10);
wherein the heater (40) is a heater coil on a heater support (28) spaced apart from the mesh screen (32), with the heater coil electrically connected to the electronic circuit; and
wherein the air flow path in the housing (10) comprises a passageway (38) in the heater support (28) and the opening is a central opening (36) extending through the liquid storage section (34) to the outlet (42).

10. The electronic cigarette of claim 9 with the air flow path further comprising one or more inlets in the housing (10) and an opening in the flow sensor (120).

11. A method of vaporizing a liquid in an electronic cigarette, the electronic cigarette comprising a mesh element (32, 52) made of a porous material, the porous material being configured to wick liquid stored in a liquid storage (34, 54), the mesh element (32, 52) being positioned between a heater (40) and the liquid storage (34, 54), and the heater (40) being spaced apart from the mesh element (32, 52), and the electronic cigarette further comprising an opening (36, 56) leading to an outlet (42); the method comprising:
conducting liquid from the liquid storage (34, 54) to the mesh element (32, 52); sensing inhalation;
providing electric current to the heater (40) in response to sensing inhalation;
heating air via the heater (40); conducting the heated air through the mesh element (32, 52);
vaporizing, by the heated air conducted through the mesh element (32, 52), liquid being wicked on or in the mesh element (32, 52); and
conducting the vaporized liquid through the opening (36, 56) leading to the outlet (42).

12. The method of claim 11 further comprising entraining the vaporized liquid into the heated air to form a mixture of vaporized liquid and heated air, and flowing the mixture through a central opening (36) in the liquid storage (34) to an outlet (42).

13. The method of claim 12 further including positioning the heater (40) within a passageway in a heater housing, and drawing ambient air into the housing (10) via an inlet in the housing (10), with the air passing through the passageway.

14. The method of claim 12 further including a passageway (38) in the heater support (28), with the central opening (36) in the liquid storage (34) having a length at least five times greater than the length of the passageway (38) in the heater support (28).

15. The method of claim 11 with the air heated to 200 to 300 °C as it is conducted through the mesh element (32, 52).

16. The method of claim 11 further comprising conducting the heated air through an annular passage (56) surrounding the liquid storage (34), after the heated air passes through the mesh element (52).

17. The electronic cigarette of claim 9 with the liquid storage section (34) including a separate component or cartridge containing a liquid.

## Patentansprüche

1. Elektronische Zigarette, umfassend:
ein Gehäuse (10), aufweisend einen oder mehrere Einlässe (18) und einen Auslass (42);
eine Flüssigkeitsaufbewahrung (35, 54) innerhalb des Gehäuses (10);
ein Maschenelement (32, 52), das die Flüssigkeitsaufbewahrung (34, 54) berührt;
und
eine Heizvorrichtung (40), die vom Maschenelement (32, 52) beabstandet ist und dazu angeordnet ist, Luft, die durch das Maschenelement (32, 52) strömt, zu erhitzen;
wobei das Maschenelement (32, 52) zwischen der Heizvorrichtung (40) und der Flüssigkeitsaufbewahrung (34, 54) angeordnet ist innerhalb eines Strömungswegs, der während des Betriebs im Gehäuse (10) ausgebildet wird;
**dadurch gekennzeichnet, dass**
der Strömungsweg durch das Gehäuse (10) während des Betriebs ausgebildet wird über den einen oder die mehreren Einlässe (18), wobei ein Durchgang die Heizvorrichtung (40) umfasst, und wobei eine Öffnung (36, 56) zum Auslass (42) führt;
das Maschenelement aus einem porösen Material hergestellt ist, wobei das poröse Material dazu ausgestaltet ist, während des Betriebs Flüssigkeit, die in der Flüssigkeitsaufbewahrung (34, 54) aufbewahrt wird, abzutragen; und
wobei das Maschenelement (32, 52) ferner dazu ausgestaltet ist, während des Betriebs zu gestatten, dass Flüssigkeit, die auf oder in dem Maschenelement (32, 52) abgetragen wird, vaporisiert wir durch erhitzte Luft, die durch das Maschenelement (32, 52) geleitet wird.

2. Elektronische Zigarette nach Anspruch 1, wobei die Heizvorrichtung (40) innerhalb eines Heizungsträgers (28) einen Luftdurchgang (38) aufweist, der in einer Linie ist mit einer Zentralöffnung (36), die sich durch die Flüssigkeitsaufbewahrung (34) erstreckt.

3. Elektronische Zigarette nach Anspruch 2, ferner enthaltend einen Kollektor (30) auf dem Heizungsträger (28), wobei der Kollektor um weniger als 4 mm vom Maschenelement (32) beabstandet ist.

4. Elektronische Zigarette nach Anspruch 2, wobei der Heizungsträger (28) ein rohrförmiges Vorderende aufweist, welches das Maschenelement (32) berührt.

5. Elektronische Zigarette nach Anspruch 1, ferner umfassend einen ringförmigen Strömungsweg (56) um das Äußere der Flüssigkeitsaufbewahrung (54).

6. Elektronische Zigarette nach Anspruch 1, wobei das Maschenelement (32, 52) ein Fasermaterial umfasst, das eine Dicke von weniger als 2 mm aufweist.

7. Elektronische Zigarette nach Anspruch 1, ferner umfassend eine Batterie (16) im Gehäuse (10), die elektrisch verbunden ist mit einem Strömungssensor (20), einer Leiterplatte (24) und der Heizspule (40).

8. Elektronische Zigarette nach Anspruch 1, wobei die Öffnung eine Zentralöffnung (36) ist, die sich durch die Flüssigkeitsaufbewahrung (34) zu einem Auslass (42) erstreckt.

9. Elektronische Zigarette nach Anspruch 1, ferner umfassend:
eine Batterie (16), die elektrisch verbunden ist mit einem Strömungssensor (120) und einer elektronischen Schaltung im Gehäuse (10);
wobei die Heizvorrichtung (40) eine Heizspule auf einem Heizungsträger (28) ist, der vom Maschensieb (32) beabstandet ist, wobei die Heizspule mit der elektronischen Schaltung elektrisch verbunden ist; und
wobei der Luftströmungsweg im Gehäuse (10) einen Durchgang (38) im Heizungsträger (28) umfasst und die Öffnung eine Zentralöffnung (36) ist, die sich durch den Flüssigkeitsaufbewahrungsabschnitt (34) zum Auslass (42) erstreckt.

10. Elektronische Zigarette nach Anspruch 9, wobei der Luftströmungsweg ferner einen oder mehrere Einlässe im Gehäuse (10) und eine Öffnung im Strömungssensor (120) umfasst.

11. Verfahren zum Vaporisieren einer Flüssigkeit in einer elektronischen Zigarette, wobei die elektronische Zigarette ein Maschenelement (32, 52) aus einem porösen Material umfasst, wobei das poröse Material dazu ausgestaltet ist, Flüssigkeit, die in einer Flüssigkeitsaufbewahrung (34, 54) aufbewahrt wird, abzutragen,
wobei das Maschenelement (32, 52) zwischen einer Heizvorrichtung (40) und der Flüssigkeitsaufbewahrung (34, 54) angeordnet ist, und wobei die Heizvorrichtung (40) vom Maschenelement (32, 52) beabstandet ist, und wobei die elektronische Zigarette ferner eine Öffnung (36, 56) umfasst, die zu einem Auslass (42) führt, wobei das Verfahren folgende Schritte umfasst:
Leiten von Flüssigkeit aus der Flüssigkeitsaufbewahrung (34, 54) zum Maschenelement (32, 52);
Erkennen der Inhalation;
Bereitstellen von elektrischem Strom an die Heizvorrichtung (4) als Reaktion auf erkannte Inhalation;
Erhitzen von Luft durch die Heizvorrichtung (40);
Leiten der erhitzten Luft durch das Maschenelement (32, 52);
Vaporisieren von auf oder in dem Maschenelement (32, 52) abgetragener Flüssigkeit durch die erhitzte Luft, die durch das Maschenelement (32, 52) geleitet wird; und
Leiten der vaporisierten Flüssigkeit durch die zum Auslass (42) führende Öffnung (36, 56).

12. Verfahren nach Anspruch 11, ferner umfassend Mitnehmen der vaporisierten Flüssigkeit in die erhitzte Luft, um eine Mischung aus vaporisierter Flüssigkeit und erhitzter Luft auszubilden, und Strömen der Mischung durch eine Zentralöffnung (36) in der Flüssigkeitsaufbewahrung (34) zu einem Auslass (42).

13. Verfahren nach Anspruch 12, ferner enthaltend Anordnen der Heizvorrichtung (40) innerhalb eines Durchgangs in einem Heizvorrichtungsgehäuse, und Lenken von Umgebungsluft in das Gehäuse (10) über einen Einlass im Gehäuse (10), wobei die Luft durch den Durchgang strömt.

14. Verfahren nach Anspruch 12, ferner enthaltend einen Durchgang (38) im Heizungsträger (28), wobei die Zentralöffnung (36) in der Flüssigkeitsaufbewahrung (34) eine Länge aufweist, die mindestens das Fünffache der Länge des Durchgangs (38) im Heizungsträger (28) ist.

15. Verfahren nach Anspruch 11, wobei die Luft auf 200 bis 300°C erhitzt wird, während sie durch das Maschenelement (32, 52) geleitet wird.

16. Verfahren nach Anspruch 11, ferner umfassend Leiten der erhitzten Luft durch einen Ringkanal (56), der die Flüssigkeitsaufbewahrung (34) umgibt, nachdem die erhitzte Luft durch das Maschenelement (52) geströmt ist.

17. Elektronische Zigarette nach Anspruch 9, wobei der Flüssigkeitsaufbewahrungsabschnitt (34) eine getrennte Komponente oder Patrone enthält, die eine Flüssigkeit beinhaltet.

## Revendications

1. Cigarette électronique, comprenant :
un boîtier (10) comportant une ou plusieurs entrées (18) et une sortie (42) ;
un réservoir de liquide (34, 54) à l'intérieur du boîtier (10) ;
un élément à mailles (32, 52) en contact avec le réservoir de liquide (34, 54) ; et
un élément chauffant (40) espacé de l'élément à mailles (32, 52) et positionné de façon à chauffer de l'air qui circule à travers l'élément à mailles (32, 52) ; l'élément à mailles (32, 52) étant positionné entre l'élément chauffant (40) et le réservoir de liquide (34, 54) à l'intérieur d'une voie d'écoulement formée, lors de l'utilisation, dans le boîtier (10) ;
**caractérisée en ce que**
la voie d'écoulement est formée, lors de l'utilisation, à travers le boîtier (10) par le biais de la ou des entrées (18), d'un passage comprenant l'élément chauffant (40), et d'une ouverture (36, 56) conduisant à la sortie (42) ;
l'élément à mailles est fabriqué en un matériau poreux, le matériau poreux étant conçu pour tirer par capillarité du liquide stocké dans le réservoir de liquide (34, 54), lors de l'utilisation ; et
l'élément à mailles (32, 52) étant en outre conçu pour permettre, lors de l'utilisation, à du liquide tiré par capillarité sur ou dans l'élément à mailles (32, 52) d'être vaporisé par de l'air chauffé guidé à travers l'élément à mailles (32, 52).

2. Cigarette électronique selon la revendication 1, l'élément chauffant (40) à l'intérieur d'un support d'élément chauffant (28) comportant un passage d'air (38) aligné avec une ouverture centrale (36) s'étendant à travers le réservoir de liquide (34).

3. Cigarette électronique selon la revendication 2, comprenant en outre un collecteur (30) sur le support d'élément chauffant (28), le collecteur étant espacé de l'élément à mailles (32) de moins de 4 mm.

4. Cigarette électronique selon la revendication 2, le support d'élément chauffant (28) comportant une extrémité avant tubulaire en contact avec l'élément à mailles (32).

5. Cigarette électronique selon la revendication 1, comprenant en outre une voie d'écoulement annulaire (56) autour de l'extérieur du réservoir de liquide (54).

6. Cigarette électronique selon la revendication 1, l'élément à mailles (32, 52) comprenant un matériau fibreux ayant une épaisseur inférieure à 2 mm.

7. Cigarette électronique selon la revendication 1, comprenant en outre une batterie (16) dans le boîtier (10), reliée électriquement à un capteur d'écoulement (20), à une carte de circuit imprimé (24) et à la spirale d'élément chauffant (40).

8. Cigarette électronique selon la revendication 1, dans laquelle l'ouverture est une ouverture centrale (36) s'étendant à travers le réservoir de liquide (34) jusqu'à une sortie (42).

9. Cigarette électronique selon la revendication 1, comprenant en outre :
une batterie (16) reliée électroniquement à un capteur d'écoulement (120) et à un circuit électronique dans le boîtier (10) ;
l'élément chauffant (40) étant une spirale d'élément chauffant sur un support d'élément chauffant (28) espacé du tamis à mailles (32), la spirale d'élément chauffant étant reliée électriquement au circuit électronique ; et
la voie d'écoulement d'air dans le boîtier (10) comprenant un passage (38) dans le support d'élément chauffant (28) et l'ouverture étant une ouverture centrale (36) s'étendant à travers la partie de réservoir de liquide (34) jusqu'à la sortie (42).

10. Cigarette électronique selon la revendication 9, la voie d'écoulement d'air comprenant en outre une ou plusieurs entrées dans le boîtier (10) et une ouverture dans le capteur d'écoulement (120).

11. Procédé de vaporisation de liquide dans une cigarette électronique, la cigarette électronique comprenant un élément à mailles (32, 52) fabriqué en matériau poreux, le matériau poreux étant conçu pour tirer par capillarité du liquide stocké dans un réservoir de liquide (34, 54),
l'élément à mailles (32, 52) étant positionné entre un élément chauffant (40) et le réservoir de liquide (34, 54), et l'élément chauffant (40) étant espacé de l'élément à mailles (32, 52), et la cigarette électronique comprenant en outre une ouverture (36, 56) conduisant à une sortie (42) ; le procédé comprenant :
le guidage de liquide du réservoir de liquide (34, 54) à l'élément à mailles (32, 52) ;
la détection d'une inhalation ;
l'alimentation de l'élément chauffant (40) en courant électrique en réponse à la détection d'une inhalation ;
le chauffage d'air par le biais de l'élément chauffant (40) ;
le guidage de l'air chauffé à travers l'élément à mailles (32, 52) ;
la vaporisation, par l'air chauffé guidé à travers l'élément à mailles (32, 52), de liquide qui est tiré par capillarité sur ou dans l'élément à mailles (32, 52) ; et
le guidage du liquide vaporisé à travers l'ouverture (36, 56) conduisant à la sortie (42).

12. Procédé selon la revendication 11, comprenant en outre l'entraînement du liquide vaporisé dans l'air chauffé pour former un mélange de liquide vaporisé et d'air chauffé, et l'écoulement du mélange à travers une ouverture centrale (36) dans le réservoir de liquide (34) jusqu'à une sortie (42).

13. Procédé selon la revendication 12, comprenant en outre le positionnement de l'élément chauffant (40) à l'intérieur d'un passage dans un boîtier d'élément chauffant, et le tirage d'air ambiant dans le boîtier (10) par le biais d'une entrée ménagée dans le boîtier (10), l'air passant par le passage.

14. Procédé selon la revendication 12, comprenant en outre un passage (38) dans le support d'élément chauffant (28), l'ouverture centrale (36) dans le réservoir de liquide (34) présentant une longueur au moins cinq fois supérieure à la longueur du passage (38) dans le support d'élément chauffant (28).

15. Procédé selon la revendication 11, l'air étant chauffé à 200 à 300 °C lorsqu'il est guidé à travers l'élément à mailles (32, 52).

16. Procédé selon la revendication 11, comprenant en outre le guidage de l'air chauffé à travers un passage annulaire (56) entourant le réservoir de liquide (34), après le passage de l'air chauffé par l'élément à mailles (52).

17. Cigarette électronique selon la revendication 9, la partie de réservoir de liquide (34) comprenant un composant séparé ou une cartouche contenant un liquide.
